# EUROPÄISCHE PATENTSCHRIFT

(11) **EP 0 753 499 B1**
(45) Veröffentlichungstag und Bekanntmachung des Hinweises auf die Patenterteilung: **13.06.2001**
(21) Anmeldenummer: 96110592.1
(22) Anmeldetag: 01.07.1996
(51) Int. Cl.: C07C 17/08, C07C 23/08

(54) **Verfahren zur Herstellung von Cyclopentylbromid**
Process for preparing cyclopentylbromide
Procédé pour la préparation du bromure de cyclopentane

(30) Priorität: 12.07.1995 DE 19525356; 14.02.1996 DE 19605402
(43) Veröffentlichungstag der Anmeldung: 15.01.1997
(73) Patentinhaber: BAYER AG, 51368 Leverkusen (DE)
(72) Erfinder: Landscheidt, Heinz, Dr., 47057 Duisburg (DE); Klausener, Alexander, Dr., 50670 Köln (DE); Stenger, Matthias, Dr., 40789 Monheim (DE); Wagner, Paul, Dr., 40597 Düsseldorf (DE)

(56) Entgegenhaltungen:
- GB-A- 1 180 037
- US-A- 3 763 250
- US-A- 3 812 212
- J. ORG. CHEM., Bd. 54, 1989, Seiten 1463-1465, XP002015515 G.A.OLAH ET AL.: "Ionic Chlorination (Bromination) of Alkanes and Cycloalkanes with Methylene Chloride(Bromide)/Antimony Pentafluoride"

## Beschreibung

Die vorliegende Erfindung betrifft ein kontinuierliches Verfahren zur Herstellung von Cyclopentylbromid durch Umsetzung von Cyclopenten mit Bromwasserstoff.

Cyclopentylbromid wird vor allem als Alkylierungsreagenz zur Übertragung des Cyclopentylrestes verwendet. Beispielsweise werden so Pharmazeutika und Agrochemikalien hergestellt. Die EP-A1-414 019 beschreibt Lipoxygenaseinhibitoren und die EP-A1-635 485 Herbizide, zu deren Herstellung jeweils Cyclopentylbromid eingesetzt wird.

Aus der Literatur sind nur wenige Verfahren zur Herstellung von Cyclopentylbromid bekannt. Offenkundig bereitet die Synthese dieser Verbindung gewisse Probleme. So läßt sich nach J. Org. Chem. 27, 624 bis 625 (1962) der durch Umsetzung von p-Toluolsulfonsäurechlorid mit Cyclopentanol erhältliche Ester mit Calciumbromid zu Cyclopentylbromid umsetzen.

Nach J. Am. Chem. Soc. 48, 1084 (1926) läßt sich Cyclopentanol durch Umsetzung mit Phosphortribromid in Cyclopentylbromid überführen.

Auch die direkte Umsetzung von Cyclopentanol mit Bromwasserstoff zu Cyclopentylbromid wurde beschrieben (J. Am. Chem. Soc. 64, 1801 bis 1802 (1942)), allerdings nur in einer Ausbeute von 70 % der Theorie.

Allen diesen Verfahren ist gemeinsam, daß von dem vergleichsweise teuren Cyclopentanol ausgegangen wird.

An Verfahren, die von billigeren und besser verfügbaren Ausgangsstoffen wie Cyclopentan oder Cyclopenten ausgehen, sind zu nennen: Die Umsetzung von Cyclopentan mit Methylenbromid und Antimonpentafluorid (J. Org. Chem. 54, 1463-1465 (1989)) und die Hydroborierung von Cyclopenten und Spaltung des intermediär erhaltenen Tricyclopentylborans mit Brom (Tetrahedron 40, 2763 bis 2784 (1988)).

Diese beiden Verfahren sind jedoch für eine technische Umsetzung ungeeignet, da teure Reagenzien eingesetzt werden müssen und große Mengen an Nebenprodukten und Abfällen anfallen, zu deren Entsorgung beträchtlicher Aufwand nötig ist.

Grundsätzlich sollte auch die Möglichkeit bestehen, Cyclopenten mit Bromwasserstoff direkt zur Reaktion zu bringen. Hierfür finden sich jedoch in der Literatur keine brauchbaren Beispiele. Vielmehr ist bei den in Frage kommenden Bedingungen in erheblichem Umfang mit unerwünschten Nebenreaktionen, beispielsweise Polymerisationen zu rechnen (siehe Houben-Weyl, Band 5/4, Seite 103 (1960)).

Es bestand daher die Aufgabe, ein wirtschaftliches Verfahren zur Herstellung von Cyclopentylbromid zu finden.

Es wurde nun gefunden, daß sich Cyclopenten mit Bromwasserstoff kontinuierlich zu Cyclopentylbromid umsetzen läßt, wenn die Reaktion in Gegenwart eines heterogenen Katalysators durchgeführt wird.

Weiterhin wurde gefunden, daß man das erfindungsgemäße Verfahren vorteilhafterweise so durchführt, daß man aus dem Reaktionsgemisch Cyclopentylbromid abtrennt und die sonstigen Anteile des Reaktionsgemisches, im wesentlichen unumgesetztes Cyclopenten, in die Reaktion zurückführt. Diese Arbeitsweise hat keine auffällig negativen Folgen hinsichtlich Umsatz, Ausbeute und Katalysatorstandzeit und eignet sich besonders für eine kontinuierliche Durchführung..

Beim erfindungsgemäßen Verfahren können die Ausgangsstoffe (Cyclopenten und Bromwasserstoff) rein oder verdünnt und in kondensiertem, teilkondensiertem oder gasförmigem Zustand über den Katalysator geleitet werden. Die Reaktionsprodukte kann man, insbesondere beim Arbeiten in der Gasphase, z.B. durch Kondensation, aus dem Produktstrom abtrennen.

In einer bevorzugten Ausführungsform des erfindungsgemäßen Verfahrens wird Cyclopenten zusammen mit einem Inertgasstrom, z.B. mit Stickstoff, Kohlendioxid oder Argon, und unter Zuspeisung von Bromwasserstoff sowie eines rückgeführten Anteils Cyclopenten dampfförmig in die Reaktionszone eingebracht.

In einer weiteren bevorzugten Ausführungsform des erfindungsgemäßen Verfahrens wird Cyclopenten, gegebenenfalls in Gegenwart eines unter Reaktionsbedingungen stabilen Verdünnungsmittels, in kondensierter Form, beispielsweise in Form einer Rieselphasenreaktion, mit Bromwasserstoff zur Reaktion gebracht, wobei die Zudosierung des Bromwasserstoffs im Gleichstrom oder im Gegenstrom zu Cyclopenten erfolgen kann.

Die erfindungsgemäße Umsetzung von Cyclopenten mit Bromwasserstoff kann z.B. bei Temperaturen zwischen -20 und +300°C, bevorzugt zwischen 0 und 150°C und besonders bevorzugt zwischen 10 und 110°C und z.B. bei Drucken zwischen 0,01 und 30 bar, bevorzugt zwischen 0,1 und 10 bar, besonders bevorzugt zwischen 0,5 und 5 bar und insbesondere bevorzugt zwischen 0,8 und 1,5 bar durchgeführt werden.

Als heterogene Katalysatoren für das erfindungsgemäße Verfahren eignen sich z.B. Metallverbindungen, die auf Trägermaterialien aufgebracht sind. Man kann auch stückige, geformte oder ungeformte Festkörper verwenden, die nach der Vermischung von Metallverbindungen mit Grundstoffen für Trägermaterialien und anschließender mechanischer Behandlung oder Formgebung erhalten werden können. Dem Fachmann sind solche Herstellungsprozesse grundsätzlich bekannt.

Als Metallverbindungen eignen sich insbesondere Metallsalze, z.B. solche von Kupfer, Eisen, Cobalt, Nickel, Chrom, Bismut, Zink, Cadmium, Vanadium, Wolfram, Quecksilber, Antimon, Aluminium und/oder Molybdän. Bevorzugt sind die Salze von anorganischen Säuren wie Fluoride, Chloride, Bromide, Sulfate und/oder Phosphate der genannten Metalle. Grundsätzlich lassen sich zur Herstellung der erfindungsgemäßen Katalysatoren auch Metallsalze einsetzen, die sich von organischen Säuren ableiten. Solche Katalysatoren wandeln sich unter den Reaktionsbedingungen im allgemeinen in die entsprechenden Bromide um.

Als Trägermaterial kommen beispielsweise Aktivkohle, Kieselgel, Siliciumdioxid, Kieselsäuren, Aluminiumoxid, Oxide und Phosphate der Seltenerdmetalle, Zeolithe, Alumosilikate, feste Heteropolysäuren wie Niobheteropolysäuren und keramische Körper in Frage.

Die Katalysatorherstellung kann z.B. nach einer der folgenden Verfahren erfolgen:
a) Man löst eines oder mehrere Metallsalze in einem Lösungsmittel, z.B. Wasser, tränkt mit der Lösung ein Trägermaterial und trocknet.
b) Man bereitet eine Metallsalzlösung wie bei a) und bringt diese durch Adsorption auf ein Trägermaterial auf.
c) Man vermischt eines oder mehrere Metallsalze, gegebenenfalls Hilfsstoffe und einen oder mehrere Grundstoffe für Trägermaterialien und überführt dieses Gemisch in stückige, geformte oder ungeformte Festkörper, z.B. in Strangpreßlinge, Zylinder, Kugeln, Ellipsoide oder Granulat. Gegebenenfalls kann sich eine Wärmebehandlung anschließen.

Bezogen auf 1 Mol Cyclopenten kann man beispielsweise 0,05 bis 5 Mol Bromwasserstoff einsetzen. Vorzugsweise liegt diese Menge bei 0,1 bis 2 Mol.

Das nach der erfindungsgemäßen Umsetzung vorliegende Reaktionsgemisch kann man beispielsweise wie folgt aufarbeiten:
Wenn bei höheren Temperaturen gearbeitet wurde, kondensiert man zunächst die z.B. bei 0°C oder niedrigeren Temperaturen kondensierbaren Anteile. Das so erhaltene Kondensat kann man dann z.B. durch Destillation auftrennen. Man erhält so Cyclopentylbromid im allgemeinen mit Umsätzen von über 45 % und in Selektivitäten von über 80 %. Im Reaktionsgemisch vorhandenes, unumgesetztes Cyclopenten kann man auch auf diese Weise abtrennen (es verbleibt dann im allgemeinen in der Gasphase) und in den Prozeß zurückführen. Im Reaktionsgemisch gegebenenfalls vorhandener unumgesetzter Bromwasserstoff kann vor oder nach der Kondensation durch eine Wäsche, z.B. mit Wasser, entfernt werden.

Das erfindungsgemäße Verfahren kann z.B. mit einer Katalysatorbelastung (GHSV) von 100 bis 10 000 h⁻¹, vorzugsweise 200 bis 5 000 h⁻¹ durchgesetzt werden.

Bevorzugt arbeitet man das Reaktionsgemisch auf, indem man das den Reaktor, z.B. einen Rohrbündelreaktor, verlassende Reaktionsgemisch einer Abtrennvorrichtung zuführt, in der Cyclopentylbromid abgetrennt wird. Bei der Abtrennvorrichtung kann es sich z.B. um eine Destillationskolonne handeln, in der Cyclopentylbromid als Sumpfprodukt abgetrennt wird. Die dabei am Kopf der Kolonne anfallende Gasphase kann man einem Kondensator zuführen. Das dort anfallende Kondensat kann man zum Teil als Rücklauf in den oberen Teil der Destillationskolonne und zum Teil in den Reaktor zurückführen. Die den Kondensator verlassende Gasphase führt man in den Reaktor zurück, wobei man einen kleinen Teil davon ausschleusen und so Nebenprodukte aus dem Verfahren herausnehmen kann.

Bei einer besonders bevorzugten, kontinuierlichen Ausführungsform des erfindungsgemäßen Verfahrens wird wie folgt gearbeitet: Der Reaktor wird bei Temperaturen im Bereich 20 bis 100°C und bei Drucken im Bereich 0,5 bis 5 bar betrieben. Ihm wird ein Gemisch zugeführt, das beispielsweise 5 bis 15 Gew.-% Cyclopenten, 60 bis 90 Gew.-% Inertgas, 2 bis 10 Gew.-% Bromwasserstoff, 0 bis 10 Gew.-% Cyclopentylbromid und 0 bis 10 Gew.-% zusammen mit Cyclopenten rückgeführte niedrigsiedende Nebenprodukte enthält. Das den Reaktor verlassende Reaktionsgemisch wird einer Destillationskolonne zugeführt, die so betrieben wird, daß man ein gasförmiges Kopfprodukt mit Temperaturen im Bereich 0 bis 100°C bei Drucken im Bereich 0,5 bis 5 bar abnehmen kann, das 0,1 bis 10 Gew.-% Cyclopenten, 65 bis 95 Gew.-% Inertgas, 0 bis 5 Gew.-% Bromwasserstoff, 5 bis 20 Gew.-% Cyclopentylbromid und 0 bis 10 Gew.-% flüchtige Nebenprodukte enthält, sowie bei Temperaturen im Bereich 50 bis 200°C ein flüssiges Sumpfprodukt, das mindestens 95 Gew.-% Cyclopentylbromid enthält. Das gasförmige Kopfprodukt wird abgekühlt und so ein Kondensat mit einer Temperatur von -50 bis +50°C erhalten, das 1 bis 80 Gew.-% Cyclopenten, 10 bis 95 Gew.-% Cyclopentylbromid, 0 bis 5 Gew.-% Bromwasserstoff und 0 bis 50 Gew.-% Nebenprodukte enthält. Dieses Kondensat wird im Verhältnis 10:1 bis 1:10 in zwei Teilströme geteilt, von denen einer als Rücklauf in den oberen Teil der Destillationskolonne und der andere in die Reaktion zurückgeführt wird. Die nach der Abkühlung des Kopfproduktes der Destillationskolonne verbleibende Gasphase enthält 75 bis 95 Gew.-% Inertgas, 0 bis 15 Gew.-% Cyclopenten, 0 bis 5 Gew.-% Bromwasserstoff, 0 bis 15 Gew.-% Cyclopentylbromid und 0 bis 10 Gew.-% Nebenprodukte. Aus dieser Gasphase wird ein Anteil von 0,5 bis 10 Gew.-% ausgeschleust, der Rest wird in die Reaktion zurückgeführt.

Das erfindungsgemäße Verfahren hat die Vorteile, daß es von einfachen, preiswerten und gut zugänglichen Ausgangsmaterialien ausgeht, Cyclopentylbromid in guten Ausbeuten und Selektivitäten liefert, keine besonderen ökologischen Aufwendungen nötig sind und wenig unbrauchbare Nebenprodukte (z.B. Polymerisate) anfallen. Es kann mit guten Ergebnissen und hoher Wirtschaftlichkeit auch kontinuierlich durchgeführt werden.

### Beispiele

### Beispiel 1

100 g Lanthanphosphat in Form von Granulat wurden mit einer Lösung von 10 g Kupfer(II)-chlorid in 50 ml Wasser getränkt. Der so erhaltene Katalysator wurde im Vakuum bei einer Temperatur von 35°C getrocknet.

In die Mitte eines sonst mit Raschigringen aus Glas befüllten Glasrohres mit einem Innendurchmesser von 3 cm wurden 20 ml des so erhaltenen Katalysators eingefüllt.

Durch eine Cyclopenten enthaltende Gaswaschflasche wurde kontinuierlich ein Stickstoffstrom von 5 Nl/h geleitet und der solchermaßen mit Cyclopenten gesättigte Gasstrom nach kontinuierlichem Hinzumischen von gasförmigem Bromwasserstoff (4 g/h) bei 40°C über den Katalysator geleitet.

Das am Austritt des Glasrohrs anfallende Reaktionsprodukt wurde durch Kühlung auf -10°C kondensiert. Eine Probe des Kondensats wurde mit verdünnter Natriumhydrogencarbonatlösung gewaschen und gaschromatographisch analysiert. Der auf eingesetztes Cyclopenten bezogene Umsatz betrug 50 %, die auf eingesetztes Cyclopenten bezogene Selektivität 90 %.

### Beispiel 2

Der Katalysator und das Reaktionsgefäß waren wie in Beispiel 1.

In das Glasrohr wurden mittels einer Pumpe kontinuierlich flüssiges Cyclopenten (12 g/h) und gasförmiger Bromwasserstoff (4 g/h) eingespeist. Die Temperatur des Katalysators betrug 50°C.

Die Aufarbeitung und Analyse erfolgte wie in Beispiel 1. Der Umsatz betrug 80 % und die Selektivität 85 %.

### Beispiel 3

In einen Rohrbündelreaktor (Abmessungen: Länge 3000 mm und Durchmesser: 300 mm) enthaltend 109 Einzelrohre mit einem Durchmesser von je 37 mm und einer Wandstärke von je 6,5 mm wurden 350 l eines analog Beispiel 1 hergestellten Katalysators eingebracht. Der Mantelraum des Reaktors wurde von einer auf 30°C gehaltenen Kühlflüssigkeit durchströmt.

Bei Normaldruck wurden dem Reaktor 309 kg/h eines auf 40°C temperierten Gemisches zugeführt, das 8,8 Gew.-% Cyclopenten, 5,8 Gew.-% Bromwasserstoff, 2,2 Gew.-% Cyclopentylbromid, 81,3 Gew.-% Stickstoff und 1,9 Gew.-% Cyclopentan enthielt.

Der den Reaktor verlassende Gasstrom wies eine Temperatur von 40°C auf und wurde in eine Kolonne (Länge: 3300 mm, Durchmesser: 300 mm) eingeleitet. Am Kopf der Kolonne war ein Wärmeaustauscher angebracht, der durch Kühlung mit Sole so einreguliert wurde, daß das hinter dem Kondensator austretende Gas und die aus dem Kondensator ablaufende Flüssigkeit je eine Temperatur von -10°C aufwiesen. Das den Kondensator verlassende Gas enthielt 4,7 Gew.-% Cyclopenten, 0,7 Gew.-% Bromwasserstoff, 1,0 Gew.-% Cyclopentylbromid, 91,7 Gew.-% Stickstoff und 2,0 Gew.-% Cyclopentan und die aus dem Kondensator ablaufende Flüssigkeit 10,6 Gew.-% Cyclopenten, 83,7 Gew.-% Cyclopentylbromid und 5,6 Gew.-% Cyclopentan. Das Gas wurde auf eine Temperatur von 25°C erwärmt und 0,65 Gew.-% davon (1,7 kg/h) kontinuierlich ausgeschleust und entsorgt. Der verbleibende Gasstrom wurde in die Reaktion zurückgeführt.

Der die Kolonne verlassende Bodenstrom (30 kg/h) wies eine Temperatur von 130°C auf und enthielt 99 Gew.-% Cyclopentylbromid.

Die aus dem Kondensator ablaufende Flüssigkeit wurde in zwei Teilströme aufgeteilt, von denen einer als Rücklauf in den oberen Bereich der Kolonne und der andere in die Reaktion zurückgeführt wurde. Das Gewichtsverhältnis der genannten Teilströme betrug 6:1 (Rücklauf in die Kolonne : Rückführung in die Reaktion).

Das so erhaltene Reaktionsprodukt Cyclopentylbromid war ohne weitere Reinigung für weitere Umsetzungen, etwa Alkylierungen, geeignet.

## Patentansprüche

1. Verfahren zur kontinuierlichen Herstellung von Cyclopentylbromid aus Cyclopenten und Bromwasserstoff, dadurch gekennzeichnet, daß man die Reaktion in Gegenwart von Metallverbindungen auf Trägermaterialien als Katalysator durchführt, wobei die Metallverbindungen Salze von Kupfer, Eisen, Cobalt, Nickel, Chrom, Bismut, Zink, Cadmium, Vanadium, Wolfram, Quecksilber, Antimon und/oder Molybdän sind und als Trägermaterialien Aktivkohle, Kieselgel, Siliziumdioxid, Kieselsäuren, Aluminiumoxid, Oxide und/oder Phosphate der Seltenerdmetalle, Zeolithe, Alumosilikate, feste Heteropolysäuren oder keramische Körper verwendet weren.

2. Verfahren nach Anspruch 1, dadurch gekennzeichnet, daß man die Ausgangsstoffe Cyclopenten und Bromwasserstoff rein oder verdünnt und in kondensiertem, teilkondensiertem oder gasförmigem Zustand über den Katalysator leitet.

3. Verfahren nach Ansprüchen 1 und 2, dadurch gekennzeichnet, daß man die Umsetzung bei Temperaturen zwischen -20 und +300°C und bei Drucken zwischen 0,01 und 30 bar durchführt.

4. Verfahren nach Ansprüchen 1 bis 3, dadurch gekennzeichnet, daß man auf 1 Mol Cyclopenten 0,05 bis 5 Mol Bromwasserstoff einsetzt.

5. Verfahren nach Ansprüchen 1 bis 4, dadurch gekennzeichnet, daß man das nach der Umsetzung vorliegende Reaktionsgemisch aufarbeitet, indem man zunächst die bei 0°C oder weniger kondensierbaren Anteile kondensiert und umgesetztes Cyclopenten in den Prozeß zurückführt.

6. Verfahren nach Ansprüchen 1 bis 5, dadurch gekennzeichnet, daß man aus dem Reaktionsgemisch Cyclopentylbromid abtrennt und die sonstigen Anteile des Reaktionsgemisches in die Reaktion zurückführt.

7. Verfahren nach Ansprüchen 1 bis 6, dadurch gekennzeichnet, daß man das den Reaktor verlassende Reaktionsgemisch einer Destillationskolonne zuführt, in der Cyclopentylbromid als Sumpfprodukt abgetrennt wird, die dabei am Kopf der Kolonne anfallende Gasphase einem Kondensator zuführt, das dort anfallende Kondensat zum Teil als Rücklauf in den oberen Teil der Destillationskolonne und zum Teil in den Reaktor zurückführt und die den Kondensator verlassende Gasphase auch in den Reaktor zurückführt, wobei man einen kleinen Teil davon auschleust.

## Claims

1. Process for the continuous preparation of cyclopentyl bromide from cyclopentene and hydrogen bromide, characterized in that the reaction is carried out in the presence of metal compounds on support materials as catalysts, the metal compounds being salts of copper, iron, cobalt, nickel, chromium, bismuth, zinc, cadmium, vanadium, tungsten, mercury, antimony and/or molybdenum, and activated carbon, silica gel, silicon dioxide, silicic acids, aluminium oxide, oxides and/or phosphates of the rare earth metals, zeolites, aluminosilicates, solid heteropolyacids or ceramic bodies are used as support materials.

2. Process according to Claim 1, characterized in that the starting materials cyclopentene and hydrogen bromide are passed in pure or diluted form and in a condensed, partially condensed or gaseous state over the catalyst.

3. Process according to Claim 1 or 2, characterized in that the reaction is carried out at temperatures between -20 and +300°C and at pressures between 0.01 and 30 bar.

4. Process according to any of Claims 1 to 3, characterized in that from 0.05 to 5 mol of hydrogen bromide is used per 1 mol of cyclopentene.

5. Process according to any of Claims 1 to 4, characterized in that the reaction mixture present after the reaction is worked up by first condensing the components condensable at 0°C or less and recirculating unreacted cyclopentene to the process.

6. Process according to any of Claims 1 to 5, characterized in that cyclopentyl bromide is separated from the reaction mixture and the other components of the reaction mixture are recirculated to the reaction.

7. Process according to any of Claims 1 to 6, characterized in that the reaction mixture leaving the reactor is fed to a distillation column in which cyclopentyl bromide is separated off as bottom product, the gas phase obtained at the top of the column is fed to a condenser, the condensate obtained there is partly recirculated as runback to the upper part of the distillation column and partly recirculated to the reactor and the gas phase leaving the condenser is also recirculated to the reactor, with a small part thereof being bled off.

## Revendications

1. Procédé pour la préparation continue du bromure de cyclopentyle à partir du cyclopentène et du bromure d'hydrogène, caractérisé en ce que l'on effectue la réaction en présence de catalyseurs consistant en dérivés métalliques sur matières de support, les dérivés métalliques étant des sels du cuivre, du fer, du cobalt, du nickel, du chrome, du bismuth, du zinc, du cadmium, du vanadium, du tungstène, du mercure, de l'antimoine et/ou du molybdène et les matières de support des charbons actifs, du gel de silice, de la silice, des acides siliciques, de l'alumine, des oxydes et/ou phosphates des métaux des terres rares, des zéolithes, des aluminosilicates, des hétéropolyacides solides ou des corps de matières céramiques.

2. Procédé selon la revendication 1, caractérisé en ce que l'on envoie les composés de départ : cyclopentène et bromure d'hydrogène, à l'état pur ou dilué et à l'état condensé, partiellement condensé ou gazeux, sur le catalyseur.

3. Procédé selon les revendications 1 et 2, caractérisé en ce que la réaction est effectuée à des températures allant de -20 à +300°C et des pressions allant de 0,01 à 30 bars.

4. Procédé selon les revendications 1 à 3, caractérisé en ce que l'on met en oeuvre 0,05 à 5 mol de bromure d'hydrogène par mole de cyclopentène.

5. Procédé selon les revendications 1 à 4, caractérisé en ce que, pour le traitement du mélange de réaction sortant, on condense d'abord les fractions condensables à 0°C ou au-dessous et on recycle le cyclopentène non converti dans les opérations.

6. Procédé selon les revendications 1 à 5, caractérisé en ce que, à partir du mélange de réaction, on sépare le bromure de cyclopentyle et on recycle les autres fractions du mélange de réaction à la réaction.

7. Procédé selon les revendications 1 à 6, caractérisé en ce que l'on envoie le mélange de réaction quittant le réacteur à une colonne à distiller dans laquelle on sépare le bromure de cyclopentyle en produit de pied, on envoie la phase gazeuse sortant en tête de colonne à un condenseur, on envoie le condensat ainsi obtenu en partie en reflux à la partie supérieure de la colonne à distiller et en partie en recyclage au réacteur et on recycle également la phase gazeuse quittant le condenseur au réacteur après en avoir évacué une petite fraction.
